# EUROPEAN PATENT APPLICATION

(11) **EP 2 063 268 A1**
(43) Date of publication of application: **27.05.2009**
(21) Application number: 07792855.4
(22) Date of filing: 22.08.2007
(51) Int. Cl.: G01N 33/543, C12Q 1/02, G01N 21/64, G01N 21/78, G01N 33/533, G01N 33/553

(54) **FLUORESCENT SEMICONDUCTOR MICROPARTICLE, METHOD FOR PRODUCTION OF THE MICROPARTICLE, FLUORESCENT LABELING AGENT FOR BIOLOGICAL SUBSTANCE USING THE MICROPARTICLE, AND BIOIMAGING METHOD USING THE MICROPARTICLE**

(30) Priority: 15.09.2006 JP 2006250714
(71) Applicant: Konica Minolta Medical & Graphic, Inc., Tokyo 191-8511 (JP)
(72) Inventor: TSUKADA, Kazuya, Hino-shi Tokyo 191-8511 (JP); GOAN, Kazuyoshi, Hino-shi Tokyo 191-8511 (JP); FURUSAWA, Naoko, Hino-shi Tokyo 191-8511 (JP)
(74) Representative: Alton, Andrew
(86) International application number: PCT/JP2007/066257
(87) International publication number: WO 2008/032535

(57) **Abstract**

Disclosed is a fluorescent semiconductor particle having a core/shell structure composed of a core particle as a semiconductor particle, and a shell layer by which the core particle is covered, wherein the core particle has a different chemical composition from that of the shell layer; the core particle has an average particle diameter of 1 - 15 nm and a specific gravity of 1.0 - 3.0; and the fluorescent semiconductor particle having the core/shell structure has a specific gravity of 0.8 - 3.2

## Description

### TECHNICAL FIELD

The present invention relates to a fluorescent semiconductor particle, a method of manufacturing the same, a biosubstance fluorescent labeling agent employing the same, and a bioimaging method thereof.

Specifically, the present invention relates to a fluorescent semiconductor particle exhibiting excellent particle diameter distribution together with dispersion stability, and further relates to a biosubstance fluorescent labeling agent useful for dynamic imaging in biology and an immunity analysis field to conduct kinetic analysis of cells and to a bioimaging method employing the same.

### BACKGROUND

In recent years, studies of nanosized semiconductor particles capable of controlling fluorescent wavelength with a particle diameter have been actively done. Since the nanosized semiconductor particles exhibit controllability of fluorescent wavelength, high photobleach, and a high degree of freedom of surface modification, they are utilized as a fluorescent marker inside or outside an organism, and studies thereof are in progress.

Particularly in recent years, the basic medical research to examine a reaction mechanism of biomolecules inside a living cell by conducting kinetic analysis further at the molecular level from qualitative assay by organism recognition, and the bioimaging research to determine biological action of viruses as well as bacteria as a disease source, and biological action of a medicine have been actively done. As specifically typified by molecular imaging, one molecule of a labeled organism substance such as a nucleus inside a cell, an endoplasmic reticulum, a golgi body, a protein, an antibody, a DNA or an RNA is bonded to one molecule or a few molecules of a fluorescent labeling agent, and luminescence via exposure to the predetermined stimulating light is detected to obtain organism information which has not been obtainable so far (such as a dynamic stage toward DNA transcription mRNA·protein formation, a cellular apoptosis dynamic stage or the like). In this case, since it is favorable to track an intrinsic dynamic stage inside an organism of organism molecules as a target, a labeling substance adsorbed or bonded to a target molecule has been desired not to inhibit movement of the target. Further, since it is pointed out that an emission amount per one specimen to be detected is small because of dynamic stage tracking of one moleculae, resulting in insufficient accuracy, further improvement of accuracy has been demanded (Patent Documents 1 - 3, for example).

Further, since an organic dye and a fluorescent protein which have been utilized for organism labeling in the past produce an insufficient emission amount, when making an effort to improve detectability (ingenuity to increase stimulating light intensity, to increase an excitation amount via focusing or the like, for example), rapid photobleach is generated, and an imbalance of detectability versus durability is provided, whereby it is pointed out that imaging is low in accuracy. As a result, a difficult problem concerning molecular imaging has been produced.

On the other hand, in order to be a labeling agent applied for organism, dispersion stability is desired to be obtained, and it is demanded for detectability that particles having excellent particle size distribution are provided to the organism with dispersion of them. According to the nature of specific gravity and so forth of semiconductor particles made of CdSe, CdTe or the like which have been utilized in the past, since a lot of dispersant (activators) should be attached onto the surface of the particles in order to sufficiently disperse them in a solution, resulting in a problem such as occurrence of rapid precipitation, whereby there appears another problem such as synthetic supply in a situation of dispersion. In order to have excellent dispersibility, a shell and a particle to which a lot of dispersant is attached inhibit a dynamic state of the target molecule via increase of the particle diameter in size, resulting in occurrence of a precipitation and formation of an aggregate. In this case, since a particle size distribution is deteriorated via increase of coarse particles, it has been pointed out that there is a problem such that accuracy tends to be largely deteriorated because of irregular detection.
Patent Document 1: Japanese Patent O.P.I. Publication No. 2003-329686
Patent Document 2: Japanese Patent O.P.I. Publication No. 2005-172429
Patent Document 3: Japanese Patent O.P.I. Publication No. 2003-524147

### DISCLOSURE OF THE INVENTION

### PROBLEMS TO BE SOLVED BY THE INVENTION

The present invention has been made on the basis of the above-described problems, and it is an object of the present invention to provide a fluorescent semiconductor particle to realize dynamic imaging in high detection accuracy, a method of manufacturing the same, a biosubstance fluorescent labeling agent employing the same, and a bioimaging method thereof.

### MEANS TO SOLVE THE PROBLEMS

The above-described problems of the present invention are solved by the following structures.

(Structure 1) A fluorescent semiconductor particle having a core/shell structure composed of a core particle as a semiconductor particle, and a shell layer by which the core particle is covered, wherein the core particle has a different chemical composition from that of the shell layer; the core particle has an average particle diameter of 1 - 15 nm and a specific gravity of 1.0 - 3.0; and the fluorescent semiconductor particle having the core/shell structure has a specific gravity of 0.8 - 3.2.

(Structure 2) The fluorescent semiconductor particle of Structure 1, wherein the specific gravity of the shell layer is adjusted by a volume ratio of the core particle to the shell layer, in such a manner that the fluorescent semiconductor particle having the core/shell structure has a specific gravity of 0.8 - 3.2.

(Structure 3) The fluorescent semiconductor particle of Structure 1 or 2, wherein the core particle has an average particle diameter of 1 - 10 nm, and the fluorescent semiconductor particle having the core/shell structure has an average particle diameter of 3 - 15 nm.

(Structure 4) The fluorescent semiconductor particle of any one of Structures 1 - 3, wherein the core particle has an average particle diameter of 1 - 5 nm, and the fluorescent semiconductor particle having the core/shell structure has an average particle diameter of 3 - 10 nm.

(Structure 5) A method of manufacturing the fluorescent semiconductor particle of any one of Structures 1 - 4, comprising the step of forming the core particle or the shell layer via a liquid phase process.

(Structure 6) A biosubstance fluorescent labeling agent comprising a surface modification compound comprising a functional group bonded to a biosubstance on a surface of the fluorescent semiconductor particle of any one of Structures 1 - 4 and a functional group bonded to the surface of the fluorescent semiconductor particle.

(Structure 7) The biosubstance fluorescent labeling agent of Structure 6, having an average particle diameter of 1 - 20 nm.

(Structure 8) The biosubstance fluorescent labeling agent of Structure 6 or 7, having an average particle diameter of 1 - 10 nm.

(Structure 9) A bioimaging method comprising the step of conducting fluorescent dynamic imaging employing the biosubstance fluorescent labeling agent of any one of Structures 6 - 8.

### EFFECT OF THE INVENTION

Through the above-described structures of the present invention, provided are a fluorescent semiconductor particle to realize an excellent particle size distribution with dispersion stability and dynamic imaging in high detection accuracy, a method of manufacturing the same, a biosubstance fluorescent labeling agent employing the same, and a bioimaging method thereof.

In addition, in the present invention, a measuring method in high sensitivity together with high accuracy can be specifically provided in a research field of one molecular imaging.

### DESCRIPTION OF THE PREFERRED EMBODIMENTS

Next, the present invention and constituent parts thereof will be described in detail.

### (Fluorescent semiconductor particle)

The fluorescent semiconductor particle of the present invention is a fluorescent semiconductor particle having a core/shell structure composed of a core particle as a semiconductor particle, and a shell layer by which the core particle is covered, wherein the core particle has a different chemical composition from that of the shell layer; the core particle has an average particle diameter of 1 - 15 nm and a specific gravity of 1.0 - 3.0; and the fluorescent semiconductor particle having the core/shell structure has a specific gravity of 0.8 - 3.2. Further, the specific gravity of the shell layer is adjusted by a volume ratio of the core particle to the shell layer, in such a manner that the fluorescent semiconductor particle having the core/shell structure has a specific gravity of 0.8 - 3.2.

Herein, "specific gravity" means a weight ratio with respect to water at 4 °C having the same volume as generally expressed.

In the present invention, the core particle has a specific gravity of 1.0 - 3.0, and preferably has a specific gravity of 1.0 - 2.5.

The fluorescent semiconductor particle of the present invention preferably has an average particle diameter of 3 - 15 nm, and more preferably has an average particle diameter of 3 - 10 nm.

In order to produce the effect, the after-mentioned bio-labeling agent preferably has a particle diameter of 1 - 20 nm, and because of this, it is specifically preferable the particle diameter is 1 - 20 nm.

Herein, "average particle diameter" is referred to as an accumulated 50% volume particle diameter measured by a laser scattering method.

In addition, as a method of detecting a shell layer thickness or a fluorescent semiconductor particle having a core/shell structure, it can be determined not only by comparing a core particle with a particle having a core/shell structure via observation with a TEM (transmission electron microscope), but also by measuring each particle diameter.

### (Core particle)

A core particle of the present invention is a constituent part to form a core portion of a fluorescent semiconductor particle having a core/shell structure of the present invention, but it is required to be composed of a semiconductor material capable of satisfying a condition of the above-described specific gravity. In addition, it may contain a slight amount of a dope material such as Ga or the like, if desired.

Various semiconductor materials are usable as the semiconductor material used for a core, according to the usage to satisfy the condition of the above-described specific gravity. Specific examples thereof include MgS, MgSe, MgTe, CaS, CaSe, CaTe, SrS, SrSe, SrTe, BaS, BaTe, ZnS, ZnSe, ZnTe, CdS, CdSe, CdTe, GaAs, GaP, GsSb, InGaAs, InP, InN, InSb, InAs, AlAs, AlP, AlSb, AlS, PbS, PbSe, Ge, Si, an admixture of these and so forth. In the present invention, a specifically preferable semiconductor material is Si.

In addition, when nanosized semiconductor particles are formed by utilizing the above-described semiconductor material and so forth, a mixture ratio and so forth should be adjusted so as to satisfy the condition of the above-described specific gravity.

In order to produce the effect of the present invention, the core of the present invention is required to have an average particle diameter of 1 - 15 nm. In addition, it becomes possible to label and detect a biomolecule having a small particle diameter by setting the average particle diameter to 1 - 10 nm. Further, labeling and dynamic imaging to one biomolecule become sufficiently possible by setting the average particle diameter to 1 - 5 nm. Accordingly, the average particle diameter of 1 - 10 nm is more preferable, and the average particle diameter of 1 - 5 nm is still more preferable.

In addition, "average particle diameter" of the core of the present invention is referred to as an accumulated 50% volume particle diameter measured by a laser scattering method.

### (Shell layer)

The shell layer of the present invention is a layer to cover the above-described core particle in a fluorescent semiconductor particle of the present invention, and is a constituent layer to form a core/shell structure, but it should be made of a semiconductor material capable of satisfying a condition of the above-described specific gravity.

Incidentally, the shell layer may not be a layer to perfectly cover the entire surface of the core particle, as long as partial exposure of the core particle with respect to the shell layer produces no problem.

Various semiconductor materials are usable as the semiconductor material used for the shell, according to the usage to satisfy the condition of the above-described specific gravity. Specific examples thereof include ZnO, ZnS, ZnSe, ZnTe, CdO, CdS, CdSe, CdTe, MgS, MgSe, GaS, GaN, GaP, GaAs, GaSb, InAs, InN, InP, InSb, AlAs, AIN, AlP, AlSb, an admixture of these and so forth.

In addition, as a favorable shell material, provided is a semiconducting material having higher band gap energy than that of a semiconducting nanosized crystalline core. In addition to the semiconducting material having higher band gap energy than that of the semiconducting nanosized crystalline core, the material suitably employed for a shell should exhibit excellent conductivity to semiconducting nanosized crystals of the core, and valence band offset. Accordingly, the conduction band is desired to be higher than a conduction band of semiconducting nanosized crystals of the core, and the valence band is desired to be lower than a valence band of semiconducting nanosized crystals of the core. A material having band gap energy in the UV region is usable as a semiconducting nanosized crystalline core to emit energy in the visible region (Si, Ge or GaP, for example) or in the near infrared region (InP, InN, PbS or PbSe, for example), and examples thereof include ZnS, GaN, and magnesium chalcogenide such as MgS, MgSe or MgTe.

A material having band gap energy in the visible region is also usable as a semiconducting nanosized crystalline core to emit energy in the near infrared region.

In the present invention, a specifically preferable semiconductor material is SiO₂ or ZnS.

### <Method of manufacturing fluorescent semiconductor particle>

Commonly known various methods are usable to prepare fluorescent semiconductor particles of the present invention.

The manufacturing method is a precipitation method as a liquid phase method, and examples thereof include a coprecipitation method, a sol-gel method, a homogeneous precipitation method and a reductive method. There are other methods such as a reversed micelle method, a supercritical hydrothermal synthesis method and so forth as the excellent method to prepare nanosized particles (refer to Japanese Patent O.P.I. Publication No. 2002-322468, Japanese Patent O.P.I. Publication No. 2005-239775, Japanese Patent O.P.I. Publication No. 10-310770 and Japanese Patent O.P.I. Publication No. 2000-104058), for example).

Usable examples of the manufacturing methods as the liquid phase method include (1) a shell method by which raw material of a semiconductor is vaporized with the first high temperature plasma generated between electrodes facing to each other, and directed under reduced pressure atmosphere through the second high temperature plasma generated via electrodeless discharge (refer to Japanese Patent O.P.I. Publication No. 6-279015, for example), (2) a method in which nanosized particles are separated and removed from an anode made of raw material of a semiconductor via electrochemical etching (refer to Published Japanese Translation of PCT International Publication No. 2003-515459, for example), a laser ablation method (refer to Japanese Patent O.P.I. Publication No. 2004-356163, for example) and so forth. Further, a method by which powder containing particles is synthesized via vapor phase reaction of raw material gas at low pressure is preferably utilized.

As a method of manufacturing the fluorescent semiconductor particle of the present invention, a manufacturing method as a liquid phase method is specifically preferable. That is, since in the present invention, a composition at which each of the core and the shell of the fluorescent semiconductor particle has the above-described specific gravity should be selected, in the case of the liquid phase method, the system is sufficiently dispersed with no appearance of particle precipitation in the dispersion after forming particles to easily form particles having the intended particle diameter together with a narrow particle diameter distribution.

### <Surface modification of fluorescent semiconductor particle>

In order to utilize the fluorescent semiconductor particle of the present invention as a labeling agent applied for organism, the fluorescent semiconductor particle surface should be modified with a surface modification compound.

The surface modification compound is preferably a compound possessing at least one functional group and at least one group bonded to a fluorescent semiconductor particle. The former group with affinity for a biosubstance is a functional group bonded to a biomolecule, and the latter group is a group capable of adsorbing onto the hydrophobic fluorescent semiconductor particle. Used may be various kinds of linkers by which surface modification compounds are connected to each other.

A group bonded to a fluorescent semiconductor particle may be a functional group bonded to a semiconductor material to form the foregoing shell layer or core particle. Accordingly, a favorable functional group is preferably selected, depending on the composition of the shell layer or the core particle. In the present invention, a thiol group is specifically preferable as the functional group.

Examples of the functional group bonded in affinity to a biosubstance include a carboxyl group, an amino group, a phosphonic acid group, a sulfonic acid group and so forth.

In addition, herein "biosubstance" means a cell, a DNA, an RNA, oligonucleotide, a protein, an antibody, an antigen, an endoplasmic reticulum, a nucleus, a golgi body and so forth.

Further, as to a method of bonding to the fluorescent semiconductor particle, a mercapto group can be bonded to the particle via adjustment of pH to be suited for surface modification. Each of an aldehyde group, an amino group and a carboxyl group is provided at the other terminal to form peptide bonding to an amino group or a carboxyl group in the organism. The similar bonding can also be made by introducing an amino group, an aldehyde group or a carboxyl group into a DNA, oligonucleotide or the like.

Specific preparation for surface modification of the fluorescent semiconductor particle can be made in accordance with methods disclosed in Dabbousi et al. J. Phys. Chem. B101: 9463 (1997); Hines et. al. J. Phys. Chem. 100: 468-471 (1996); Peng et al. J. Am. Chem. Soc. 119: 7019-7029 (1997); and Kuno et al. Phys. Chem. 106: 9869 (1997).

### (Biosubstance fluorescent labeling agent and bioimaging method employing biosubstance fluorescent labeling agent)

The fluorescent semiconductor particle is suitable for a biosubstance fluorescent labeling agent in accordance with the following description. Further, the biosubstance fluorescent labeling agent of the present invention is added into a living cell or organism having a target (tracking) substance to have one adsorbed or bonded to the target substance, and the adelphus or adsorbent is exposed to stimulating light having the predetermined wavelength, whereby fluorescence having the predetermined wavelength, generated from the fluorescent semiconductor particle depending on the stimulating light, is detected to easily conduct fluorescent dynamic imaging for the above-described target (tracking) substance. That is, the biosubstance fluorescent labeling agent of the present invention can be utilized for a bioimaging method (a technique by which a biomolecule constituting a biosubstance and its dynamic phenomenon are visualized).

### BIOSUBSTANCE FLUORESCENT LABELING AGENT

Next, the biosubstance fluorescent labeling agent and the related art will be described in detail.

The fluorescent semiconductor particle which has been surface-modified in the present invention (hereinafter, referred to also as "surface modification semiconductor particle") can be bonded to an affinity molecule serving as the first member of a bonding pair with a functional group in a surface modification compound. For example, an ionizable group existing in a hydrophilic structure part of the surface modification compound can provide a bonding means to the affinity molecule.

In addition, a suitable method by which a molecule and a molecular segment are bonded to the affinity molecule is described in Hermanson, Bioconjugate Techniques (Academic Press, NY, 1996).

"Adelphus" with such the surface modification semiconductor particle via the affinity molecule is usable to detect the presence and/or the amount of a biosubstance, that is, a biological compound and a chemical compound; interaction in a biological system or a biological process; change in the biological process; or change of the structure of the biological compound. That is, in the case of bonding to the surface modification semiconductor particle, the affinity molecule is capable of detecting the biological process or response via interaction with a biological target serving as the second member of the bonding pair, or of changing the biological molecule or process.

As a preferable situation, the interaction of the affinity molecule and the biological target can be accompanied with specific bonding, and can be accompanied with covalent bonding, noncovalent bonding, hydrophobicity, hydrophilicity, and van der waals or magnetic interaction. Further, the affinity molecule is possible to physically interact with the biological target.

The affinity molecule bonded to the surface modification semiconductor particle is possible to occur in nature, or to be chemically synthesized, and the desired physical, chemical or biological property possessed by that can be selected.

As such the property, provided are covalent bonding and noncovalent bonding and so forth with a protein, a nucleic acid, a signal-transducing molecule, a prokaryotic cell or an eukaryotic cell, a virus, organelle inside a cell or any other biological compound, but the present invention is not limited thereto.

As other properties of such the molecule, provided are ability to affect the biological process (for example, cell cycle, blood clotting, cell death, transcription, translation, signal-transducing, DNA damage or DNA break, radical production, radical removal or the like), ability to change a structure of the biological compound (for example, cross-linkage, protein cleavage, radical injury or the like) and so forth, but the present invention is not limited thereto.

In a preferable embodiment, a surface modification semiconductor particle adelphus contains semiconductor particles emitting light of an adjustable wavelength, and is bonded to a nucleic acid. The foregoing bonding may be direct bonding or indirect bonding. The nucleic acid may be any of a ribonucleic acid, a deoxyribonucleic acid and a dideoxyribonucleic acid, or any derivative thereof, and a combination of these. The nucleic acid can also be oligonucleotide having an arbitrary length. This oligonucleotide may have a single strand, a double strand, a triple strand or a higher configuration [for example, a holiday junction, a cyclic single-stranded DNA, a cyclic double-stranded DNA and a DNA cube {refer to Seeman, Ann. Rev. Biophys. Biomol. Struct. 27: 225248 (1998)}].

A specifically preferable embodiment to use a semiconductor particle adelphus of the present invention is of detection and/or quantitative determination of a nucleic acid as described below; (a) virus nucleic acid, (b) bacterial nucleic acid, and (c) intended sequence concerning a human body (for example, single-stranded nucleotide polymorphism). The fluorescent semiconductor particle adelphus can contain the fluorescent semiconductor particle bonded to each of nucleotide, deoxynucleotide, dideoxynucleotide, any derivative thereof, and a combination of these, and is utilized for DNA polymerization reaction {for example, determination of a DNA sequence, reverse transcription of RNA to DNA, and polymerase chain reaction (PCR)}.

As nucleotide, provided are monophosphate, diphosphate, triphosphate and a cyclic derivative {for example, cyclic adenine-phosphoric acid (cAMP)}.

The other embodiment to use the fluorescent semiconductor particle bonded to a nucleic acid includes fluorescent in situ hybridization (FISH). In this preferable embodiment, the fluorescent semiconductor particle is bonded to oligonucleotide designed to be hybridized in specific sequence in vivo. As to the hybridization, a fluorescent semiconductor particle tag is utilized to visualize a desired position of DNA sequence in a cell. For example, intracellular localization of a gene in which the DNA sequence is partially or completely known can be determined via FISH.

Any DNA or RNA in which the sequence is partially or completely known is possible to be visually labeled by FISH. For example, messenger RNA (mRNA), telomeric DNA, highly repeated DNA sequence and another noncoding DNA sequence are possible to be labeled by FISH, without limiting the scope of the present invention.

The fluorescent semiconductor particle adelphus also includes the surface modification semiconductor particle, as described in the present application, obtained via bonding to a molecule or a reagent to detect a biological compound (organella, lipid, phospholipid, an aliphatic acid, sterol, a cell membrane, a signal-transducing molecule, a receptor and an ion channel, for example).

The adelphus is also possible to be utilized for detection of the cell configuration and fluid flow; viability, proliferation and functioning of the cell; endocytosis and exocytosis disclosed in Betz et al., Curr. Poin. Neurobiol. 6(3): 367-71}; and reactive oxygen species (superoxide, nitrogen monoxide, hydroxyradical, oxygen radical, for example).

It has been found out that the biosubstance fluorescent labeling agent (fluorescent semiconductor particle adelphus) is also useful for various other biological and nonbiological applications, and in this case, a fluorescent marker, specifically a fluorescent marker is typically used. For example, "Haugland, R.P.Handbook of Fluorescent Probes and Research Chemicals (Molecular Probes, Eugene, OR. the 6^{th} edition (1996), Website www.probes.com" can be used as reference.

Examples of the region in which the biosubstance fluorescent labeling agent of the present invention is useful include fluorescent immunocytochemistry, a fluorescent microscopy method, DNA sequence analysis, fluorescent in situ hybridization (FISH), fluorescent resonance energy transfer (FRET), flow cytometry (fluorescence activated cell sorter; FACS), diagnosis assay in a biological system and so forth, but the present invention is not limited thereto.

Concerning discussion with respect to usefulness of the adelphus having nanosized crystals in the above-described region, WO No. 00/17642 of Bawendi et al. can be used as reference.

Hereinbefore, as described above, immunostaining employing a fixed cell, cell observation, real-time tracking in receptor-ligand interaction (low molecular drug), one molecule fluorescent imaging and so forth are provided as the application range of the present invention.

### EXAMPLE

Next, the present invention will be described in detail referring to examples, but the present invention is not limited thereto.

### Example 1

### (Preparation of Si core particle, and Si core/SiO₂ shell particle)

### <HF etching method>

When Si fluorescent semiconductor particles (hereinafter, referred to also as "Si semiconductor particles" or "Si core particles") are prepared by dissolving SiO₂ (x = 1.999) having been subjected to a heat treatment in a hydrofluoric acid, SiOₓ (x = 1.999) layered on a silicone wafer via plasma CVD is first annealed at 1,100 °C for one hour under an inert gas atmosphere to precipitate Si semiconductor particles (crystals) in a SiO₂ film.

Next, this silicone wafer is treated at room temperature with an approximately 1% aqueous hydrofluoric acid solution to remove the SiO₂ layer, and Si semiconductor particles in several nm size, coagulated on the liquid surface are collected. In addition, via this hydrofluoric acid treatment, a dangling bond (non-bonding means) of a Si atom on the semiconductor particle (crystal) surface is terminated by hydrogen, whereby the Si crystal is stabilized. Thereafter, the collected Si semiconductor particle surface undergoes natural oxidation in oxygen atmosphere or thermal oxidation by heat to form a shell layer made of SiO₂ around a Si semiconductor particle as a core.

### (Anodic oxidation method)

Further, when the Si semiconductor particle is prepared via anodization of a p-type silicon wafer, electricity is first applied to the p-type silicone wafer and platinum as facing electrodes at 320 mA/cm² for one hour in a solution in which hydrofluoric acid (46%), methanol (100%) and hydrogen peroxide water (30%) are mixed at a content ratio of 1 : 2 : 2 to precipitate the Si semiconductor particle (crystal). The surface of the Si semiconductor particle obtained in this way undergoes natural oxidation in oxygen atmosphere or thermal oxidation by heat to form a shell layer made of SiO₂ around a core composed of Si crystals.

### (Preparation of Si core/ZnS shell particle)

The resulting Si core particles described above were dispersed in pyridine, and maintained at 100 °C. Separately, Zn(C₂H₅)₂, {(CH₃)₃Si}₂S and P(C₄H₉)₃ were slowly mixed in an argon gas atmosphere via application of ultrasonic waves while adjusting pH.

The resulting was dropped in the pyridine dispersion. Temperature was appropriately controlled after the addition, and the system was slowly stirred for 30 minutes while keeping the pH constant (pH of 8.5 at 25 °C). The resulting was centrifuged to collect precipitated particles. Si and ZnS were identified via element analysis of the resulting particles, and it was found out via XPS analysis that ZnS covered the surface of Si.

### (Comparative particle formation 1: preparation of CdSe core/SiO₂ shell particle)

After charging 0.14 g of cadmium acetate and 5.0 g of trioctyl phosphine oxide (TOPO) in an egg plant flask, and filling the system with argon, the system was heated to a predetermined temperature of 150 - 250 °C. Into this solution, quickly added was 1.44 cm³ of tri-n-octyl phosphine solution, in which selenium was dissolved, so as to give a concentration of 25 mg/cm³ while heavily stirring, and the system was further stirred for one hour to obtain TOPO-stabilized CdSe (hereinafter, referred to as "TOPO/CdSe"). When TOPO/CdSe was synthesized at each of 200 °C and 150 °C, absorption spectrum rise wavelengths of the CdSe semiconductor particles were 650 nm and 610 nm, respectively, and average particle diameters thereof were 5.5 nm and 4.5 nm, respectively. The surface of the CdSe semiconductor particle was modified with 3-mercaptopropyltriethoxysilane by using this TOPO/CdSe powder, and then hydrolysis was further carried out to obtain a CdSe core/silica shell structural body (hereinafter, referred to as "CdSe/SiO₂") in which a silica thin film was formed on a core particle surface.

The resulting core/shell structural body in a photodissolution solution was exposed to monochromatic light, and the cadmium selenide (CdSe) semiconductor particle inside the core/shell structural body was subjected to a size selection photoetching treatment to obtain a fluorescent semiconductor particle composed of a core/shell structural body in which a particle diameter of the cadmium selenide (CdSe) semiconductor particle was reduced to 3.5 nm.

### (Comparative particle formation 2: preparation of CdSe core/ZnS shell particle)

After charging 0.14 g of cadmium acetate and 5.0 g of trioctyl phosphine oxide (TOPO) in an egg plant flask, and filling the system with argon, the system was heated to a predetermined temperature of 150 - 250 °C. Into this solution, quickly added was 1.44 cm³ of tri-n-octyl phosphine solution, in which selenium was dissolved, so as to give a concentration of 25 mg/cm³ while heavily stirring, and the system was further stirred for one hour to obtain TOPO-stabilized CdSe (hereinafter, referred to as "TOPO/CdSe").

The resulting CdSe core particles described above were dispersed in pyridine, and maintained at 100 °C. Separately, Zn(C₂Hs)₂, {(CH₃)₃Si}₂S and P(C₄H₉)₃ were slowly mixed in an argon gas atmosphere.

The resulting was dropped in the pyridine dispersion. Temperature was appropriately controlled after the addition, and the system was slowly stirred for 30 minutes while keeping the pH constant (pH of 8.5 at 25 °C). The resulting was centrifuged to collect precipitated particles. InP and ZnS were identified via element analysis of the resulting particles, and it was found out via XPS analysis that ZnS covered the surface of CdSe.

The particle diameters of the core particle and the core/shell particle prepared as described above were measured with Zetasizer ZS, manufactured by SYSMEX Corporation, and the specific gravity was measured with SGM-6, manufactured by Mettler-Toledo International Inc. Results are shown in Table 1.

### (Introduction of modification functional group)

When a biosubstance was labeled with the above-described fluorescent semiconductor particle, functional groups or the like bonded to each other should be introduced into at least one of the particle and the biosubstance, and the following was carried out as described below.

### (Introduction of modification functional group into Si core/SiO₂ shell particle)

A carboxyl group is introduced into the fluorescent semiconductor particle via bonding of melcapto group (SH group)-to-melcapto group (SH group).

First, the above-described Si core particles are dispersed in 30% of hydrogen peroxide water for 10 minutes to hydroxylate the crystalline surface. Next, a solvent is replaced by toluene, and mercaptopropyltriethoxysilane is added in an amount of 2% of toluene to silanaize SiO₂ on the outermost surface of a Si core particle spending about 2 hours, and also to introduce a mercapto group. Subsequently, the solvent is replaced with pure water and a buffering salt is added. Further, 11-mercaptoundecanoic acid in which a mercapto group is introduced at a terminal is added in an appropriate amount, and resulting is stirred for 3 hours to combine the Si core particle with the 11-mercaptoundecanoic acid. In the present invention, provide is an example in which a modification group bonded in affinity to organism is introduced. This is designated as label A.

### (Introduction of modification functional group into Si core/ZnS shell particle)

The resulting Si core/ZnS shell particles described above are dispersed in a buffering salt solution, and the 11-mercaptoundecanoic acid is added in an appropriate amount and the resulting is stirred at a proper temperature for 2 hours to combine the mercapto group with the particle surface. By this, a carboxyl group is introduced onto the surface. This is designated as label B.

### (Introduction of modification functional group into CdSe core/SiO₂ shell particle)

The 11-mercaptoundecanoic acid is bonded to the surface by the same method as that of labeling A to introduce a carboxyl group. This is designated as label C.

### (Introduction of modification functional group into CdSe core/ZnS shell particle)

The 11-mercaptoundecanoic acid is bonded to the surface by the same method as that of labeling B to introduce a carboxyl group. This is designated as label D.

### <Fluorescent intensity analysis>

As to each labeling, light having a wavelength of 405 nm was utilized as stimulating light to evaluate fluorescent intensity employing a fluorescent spectrometer FP-6500, manufactured by TASCO Corporation. Those values were described as the relative value when label A was set to 100 as reference, and results were shown in Table 1.

### <Staining analysis to be ingested into cell>

The resulting label described above was mixed in isoconcentration with ovine serum albumin (SSA) in advance, and resulting was ingested into a Vero cell. After culture at 37 °C for 2 hours, a trypsin treatment was conducted to have 5% FMS/DMEM to be floated again, and to be disseminated in a glass bottom culture dish. A cell having been cultivated overnight at 37 °C was fixed with 4% formalin, and the nucleus was stained with DAPI to conduct fluorescent observation employing a confocal laser scanning microscope (excited at 405 nm).

The accumulating situation of cellular cytoplasm in the present label into endosome was evaluated in concentration depending on fluorescent intensity and in a dispersion state. That is, when a migration efficiency, in which the present label is ingested into a cell, migrated to endosome, and accumulated, is high, fluorescent intensity in endosome is high, and a distributional area thereof is large. On the other hand, when the migration efficiency is low via ingestion under the influence of the particle diameter, specific gravity and so forth, the fluorescent intensity is low, and the distributional area is small. The situation of this observation is described in Table 1.

Results obtained via staining after labeling with Texas red were also described in Table 1.

As is clear from the results shown in Table 1, it is to be understood that labels A and B of the present invention exhibit excellent migration efficiency to be ingested into a cell, and are suitable for cell imaging observation. Further, it can be also understood that they exhibit higher fluorescent intensity and durability in comparison to a dye, and are optimally suitable for imaging.

**Table 1**

| Label kind | Core particle diameter of (nm) | Specific gravity of core | Specific Gravity of core/shell | Fluorescent Intensity (Relative value) | Cell staining observation | Remarks |
|---|---|---|---|---|---|---|
| A | 2.0 | 2.3 | 2.2 | 100 (Reference value) | *1 | Inv. |
| B | 2.0 | 2.3 | 3.0 | 95 | *2 | Inv. |
| C | 4.5 | 7.5 | 7.2 | 80 | *3 | Comp. |
| D | 4.5 | 7.5 | 7.8 | 80 | *4 | Comp. |
| Dye | - | - | - | 20 | *5 | Comp. |

| | | | | | | |
|---|---|---|---|---|---|---|
| *1: The fluorescent area is large, and the fluorescent intensity is high. *2: The fluorescent area is slightly smaller than that of A, but the fluorescent intensity is high. *3: The fluorescent area is one-third of that of A, and the fluorescent intensity is also about one-half of that of A. *4: The fluorescent area is one-third of that of A, and the fluorescent intensity is also about one-third of that of A. *5: The fluorescent area is largely distributed, but the fluorescent intensity is low, fluorescence has hardly been observed after observation for 30 seconds. Inv.: Present invention, Comp.: Comparative example | | | | | | |

### Example 2

Avidin modification was conducted employing the core/shell particle prepared in Example 1.

Si core/SiO₂ shell particles are reacted in a mixture solution of a concentrated sulfuric acid and hydrogen peroxide water in a content ratio of 3 : 1 to hydroxylate the crystalline particle surface. After washing, aminopropyltriethoxysilane is reacted with hydroxyl groups on the outermost surface of the particle in a mixture solution of water and ethanol in a content ratio of 1 : 1 to introduce an amino group into the particle. After the reaction, washing is conducted, and a solvent is replaced by acetone to add a maleic acid so as to give a concentration of 1 mol/litter. Spending approximately 30 - 45 minutes, heating nearly to the boiling point, and refluxing are conducted to combine the amino group in the aminopropyltriethoxysilane with the carboxyl group in the maleic acid. After the reaction, washing is conducted, and the resulting is dispersed in a mixture solution of 867 mM of an aqueous N-hydroxy succinimide solution and 522mM of an aqueous N-(3-dimethylaminopropyl)-N'-ethylcarbodiimide-hydrochloride solution in a content ratio of 1 : 1 to conduct reaction for about 10 minutes. After the reaction, washing is conducted, and after reacting with 2.21 µM of avidin in an aqueous solution at room temperature, 1 mM of ethanol amine is added while stirring at room temperature for 10 minutes. By this, avidin is bonded to the particle. This is designated as label A-2.

### <Introduction of avidin into Si core/ZnS shell particle>

After introducing an amino group onto the surface via bonding to the particle with a mercapto group by using 3-mercapto-1-aminopropane, steps were conducted similarly to the case of the above-described A-2 to obtain avidin bonding label B-2.

### <Introduction of avidin into CdSe core/SiO₂ shell particle>

Steps were conducted similarly to the case of the above-described A-2 to obtain avidin bonding label C-2.

### <Introduction of avidin into CdSe core/ZnS shell particle>

Steps were conducted similarly to the case of the above-described B-2 to obtain avidin bonding label D-2.

### <Immunostaining>

GTP degradative enzyme Pan serving as nuclear membrane pore transportation was tried to be stained with each of the above-described labels. GTP degradative enzyme Pan and a label were mixed, and the resulting was ingested into a Vero cell. After cultivating the cell overnight at 37 °C, fluorescent observation was conducted employing a confocal laser scanning microscope (excited at 405 nm). The staining situations of the nucleus are described in Table 2. When observed fluorescent intensity is high, and the area is large, the label conjugate exhibits high migration efficiency, which shows a feature of the label in the present invention. When staining is hardly observed, the staining hardly observed shows that no migration is generated because of precipitation and so forth since specific gravity is too large.

**Table 2**

| Label kind | Core particle diameter (nm) | Specific gravity of core | Specific gravity of core/shell | Label fluorescent intensity (Relative value) | Cell staining observa tion | Remarks |
|---|---|---|---|---|---|---|
| A-2 | 2.0 | 2.3 | 2.2 | 100 (Refer-ence value) | *1 | Inv. |
| B-2 | 2.0 | 2.3 | 3.0 | 94 | *2 | Inv. |
| C-2 | 4.5 | 7.5 | 7.2 | 82 | *3 | Comp. |
| D-2 | 4.5 | 7.5 | 7.8 | 83 | *4 | Comp. |
| Dye | - | - | - | 20 | *5 | Comp. |

| | | | | | | |
|---|---|---|---|---|---|---|
| *1: The fluorescent area is large, and the fluorescent intensity is high. *2: The fluorescent area is slightly smaller than that of A-2, but the fluorescent intensity is high and nearly equal to that of A-2. *3: The fluorescent area is one-third of that of A-2, and the fluorescent intensity is also about one-third of that of A. *4: The fluorescent area is one-fourth of that of A, and the fluorescent intensity is also about one-fourth of that of A. *5: The fluorescent area is largely distributed, but the fluorescent intensity is low, fluorescence has hardly been observed after observation for 30 seconds. Inv.: Present invention, Comp.: Comparative example | | | | | | |

As is clear from Table 2, it is to be understood that the label of the present invention is suitable for dynamic imaging. That is, it is also to be understood that depiction in dynamic imaging of the biosubstance can be largely improved.

## Claims

1. A fluorescent semiconductor particle having a core/shell structure composed of a core particle as a semiconductor particle, and a shell layer by which the core particle is covered,
wherein the core particle has a different chemical composition from that of the shell layer; the core particle has an average particle diameter of 1 - 15 nm and a specific gravity of 1.0 - 3.0; and the fluorescent semiconductor particle having the core/shell structure has a specific gravity of 0.8 - 3.2.

2. The fluorescent semiconductor particle of Claim 1,
wherein the specific gravity of the shell layer is adjusted by a volume ratio of the core particle to the shell layer, in such a manner that the fluorescent semiconductor particle having the core/shell structure has a specific gravity of 0.8 - 3.2.

3. The fluorescent semiconductor particle of Claim 1 or 2,
wherein the core particle has an average particle diameter of 1 - 10 nm, and the fluorescent semiconductor particle having the core/shell structure has an average particle diameter of 3 - 15 nm.

4. The fluorescent semiconductor particle of any one of Claims 1 - 3,
wherein the core particle has an average particle diameter of 1 - 5 nm, and the fluorescent semiconductor particle having the core/shell structure has an average particle diameter of 3 - 10 nm.

5. A method of manufacturing the fluorescent semiconductor particle of any one of Claims 1 - 4, comprising the step of:
forming the core particle or the shell layer via a liquid phase process.

6. A biosubstance fluorescent labeling agent comprising a surface modification compound comprising a functional group bonded to a biosubstance on a surface of the fluorescent semiconductor particle of any one of Claims 1 - 4 and a functional group bonded to the surface of the fluorescent semiconductor particle.

7. The biosubstance fluorescent labeling agent of Claim 6, having an average particle diameter of 1 - 20 nm.

8. The biosubstance fluorescent labeling agent of Claim 6 or 7, having an average particle diameter of 1 - 10 nm.

9. A bioimaging method comprising the step of:
conducting fluorescent dynamic imaging employing the biosubstance fluorescent labeling agent of any one of Claims 6 - 8.
